# EUROPEAN PATENT APPLICATION

(11) **EP 1 262 142 A2**
(43) Date of publication of application: **04.12.2002**
(21) Application number: 02010616.7
(22) Date of filing: 10.05.2002
(51) Int. Cl.: A61B 5/00

(54) **Remote health care service, monitoring terminal and program for use in the terminal**

(30) Priority: 30.05.2001 JP 2001162450
(71) Applicant: Denso Corporation, Kariya-city, Aichi-pref., 448-0029 (JP)
(72) Inventor: Teraura, Nobuyuki, Kariya-city, Aichi-pref., 448-0029 (JP); Konishi, Kiyokazu, Kariya-city, Aichi-pref., 448-0029 (JP)
(74) Representative: Winter, Brandl, Fürniss, Hübner, Röss, Kaiser, Polte Partnerschaft

(57) **Abstract**

A remote care service system comprises the care center (attended by a care worker), a first-aid center, and the plurality of home subsystems, each comprising a relay terminal and at least one wireless care recipient monitoring terminal disguised as an attachment-arousing and/or familiar article. The care and first-aid centers and the wireless terminals are accessible to a communication network such as the Internet. The relay terminal relays a communication between an associated wireless terminal and the center equipment. The wireless terminal tries an interactive communication with the care recipient by using a predetermined phrase to judge whether the care recipient is normal. If not, then the wireless terminal sends an abnormality report to the first-aid center, which responsively dispatches emergency men to the care recipient's home. Judgment the care recipient's health condition interactively with the care recipient enhances the reliability of the judgment.

## Description

### BACKGROUND OF THE INVENTION

### 1. Field of the Invention

The invention relates to a remote care service system for monitoring health conditions of a care recipient from a remote care center.

### 2. Description of the Prior Art

Family makeups are changing from larger families of not only parent-and-child generations but also more generations to smaller families of only a married couple and, at most, their children, while the average life span is becoming longer. This results in a continuous increase in the number of elder persons living alone. How to take care of the aged is one of serious social problems.

U.S. Pat. (USP) No. 5,544,649 entitled Ambulatory Patient Health Monitoring Techniques Utilizing Interactive Visual Communication is provide with cameras at the patient's remote location and at the central station such that the patient and the health care worker are in interactive visual and audio communication. Various medical condition sensing and monitoring equipment are placed in the patient's home, depending on the particular medical needs of the patient. The patient's medical condition is measured or sensed in the home and the resulting data is transmitted to the central station for analysis and display.

Japanese Unexamined Patent Publication No. 10-234796 discloses a home nursing support system that enables a support center to observe a patient through a TV telephone function on a monitoring occasion or in emergency while protecting the patient's privacy.

Japanese Unexamined Patent Publication (JUPP) No. 08-50692 discloses a detection system for abnormality of person requiring care. The system measures the number and the positions of persons and two-dimensional temperature distribution at a predetermined interval; detects whether a care recipient is normal based on the measured information; and, if not, inform a predetermined destination of the abnormality of the care recipient.

However, in USP No. 5,544,649 and JUPP No. 10-234796, the judgment on whether the care recipient is normal is made by a care worker in charge analyzing received information at the care center. For this reason, the care center can monitor only a limited number of care recipients, providing less efficient remote care service.

Since the detection system provided at each care recipient cite makes the judgment on whether the care recipient is normal in JUPP No. 08-50692, the predetermined destination can serve a larger number of care recipients, providing more efficient remote care service. However, the judgment is made only based on physically measured information. There still exists a room for improving the accuracy of the judgment on the care recipient's medical or health condition.

Therefore, it is an object of the invention to provide techniques for providing remote care service to an increased number of care recipients on the basis of a higher accuracy judgment on the care recipient's health condition at home.

### SUMMARY OF THE INVENTION

According to an aspect of the invention, a remote care service technique for providing a care service to each of a plurality of care recipients remote from a care center is provided. The remote care service system comprises the care center (attended by a care worker), a first-aid center, and the plurality of home subsystems. Each home subsystem comprises a relay terminal and at least one wireless terminal (or care recipient monitoring device) disguised as any attachment-arousing and/or familiar article. The care center, the first-aid center and the wireless terminals are accessible to a communication network such as the Internet. In each home, the relay terminal relays a communication between one of the wireless terminals and the center equipment. The wireless terminal tries an interactive communication with the care recipient by using a first kind of phrase to judge whether the care recipient is normal in health condition. If the care recipient is abnormal, then the wireless terminal sends an abnormality report to the first-aid center equipment through the relay terminal. The first-aid center equipment responsively dispatches emergency men to the care recipient's home. Since the judgment of the care recipient's health condition is made interactively with the care recipient, the reliability of the judgment is enhanced.

In the judging process, it is considered whether the care recipient is absent in a monitoring space of the care recipient monitoring device for more than a predetermined period of time.

The interactive communication may include addressing the care recipient with a predetermined phrase at a predetermined time.

In the judging process, if the care recipient nether orally answers to said predetermined phrase nor move at all, then the wireless terminal judges that the care recipient is abnormal.

In the judging process, if in response to the predetermined phrase the care recipient gives a voiced response different from one or more response associated with the predetermined phrase, then the wireless terminal judges that the care recipient is abnormal.

The care center includes first speech communication means for having a conversation with a person over the communication network. The wireless terminal further comprises second speech communication means for having a conversation with the care worker through the relay terminal, the communication network and the first speech communication means. If the care recipient is absent in a monitoring space of the wireless terminal for more than a predetermined period of time, then the wireless terminal enters into an operation mode that enables the care worker to have a conversation with the care recipient.

In one embodiment of the invention, the wireless terminal further comprises means for moving to shift the position of the wireless terminal, a CCD camera for taking in an image of a monitor space of the wireless terminal and image recognizer for recognizing the care recipient and obstacles within the home in the image. If the care recipient is absent for more than a predetermined period of time, the wireless terminal searches the care recipient's home for the care recipient by using the image recognizer. In response to a success in the search, the wireless terminal tries an interactive communication with the care recipient by using a second kind of phrase to judge whether the care recipient is normal in health condition. If the care recipient is abnormal, then the wireless terminal sends an abnormality report to the first-aid center equipment through the relay terminal.

The first-aid center may include a speech communication portion for having a conversation with a person over the communication network; and a video monitoring portion for viewing the image. The first-aid center is attended by an attendant. Preferably, the wireless terminal is so configured as to enable the care recipient to have a conversation with the care worker through the relay terminal, the communication network and the speech communication portion of the first-aid center and to send the image to the video monitoring portion through the relay terminal and the communication network. In the abnormality reporting, the wireless terminal may enter into an operation mode that enables the attendant to have a conversation with the care recipient while viewing the image.

The remote care service system may further include a care center attended by a care worker. The care center comprises a first speech communication portion for having a conversation with a person over the communication network; and a video monitoring portion for viewing the image. The wireless terminal preferably further comprises a second speech communication portion for having a conversation with the care worker through the relay terminal, the communication network and the first speech communication portion; and portion for sending the image to the video monitoring portion through the relay terminal and the communication network. In response to a failure in the above-mentioned searching for the care recipient, the wireless terminal enters into an operation mode that enables the care worker to control the wireless terminal to search the house for the care recipient while viewing the image in a state capable of having a conversation with the monitor space of the wireless terminal.

The above-described features and their modifications may be combined in any suitable manner to constitute a remote care service system.

According to another aspect of the invention, there is provided a care recipient monitoring device for use in a remote care service system comprising a center equipment capable of connecting with a communication network and a home subsystem which is provided in each home of a plurality of care recipients remote from the center equipment to provide care service to each of the care recipients. The care recipient monitoring device is included in each home subsystem which further comprises relay terminal for relaying a communication between the care recipient monitoring device and the center equipment. The care recipient monitoring device comprises a portion, constituting an enclosure of the care recipient monitoring device, for disguising the care recipient monitoring device as an article such as gives a good impression to the care recipient; a radio portion for effecting a radio communication with the relay terminal; a judging portion for judging whether the care recipient is normal through interaction with the care recipient; and a reporting portion, responsive to a judgment that the care recipient is abnormal, for sending an abnormality report to the center equipment through the radio means and the relay terminal so as to cause the center equipment to dispatch emergency men to the care recipient's home.

According to further aspect of the invention, there is provided a computer program recorded on a computer-readable medium and designed to operate a computerized device so as to provide a care service to a care recipient in a home remote from a center equipment in cooperation with a relay terminal installed in the home. The relay terminal relays a communication between the computerized device and the center equipment which are linked with a communication network. The center equipment serves a multiplicity of care recipients. The computerized device comprises a computer portion for controlling the entirety of the computerized device; a radio portion for effecting a radio communication with the relay terminal; a speech recognizing portion for recognizing a speech uttered by the care recipient; and a speech synthesizing portion responsive to a code from the computer portion for outputting one of predetermined synthesized speech associated with the code. The computer program comprises the steps of through the speech synthesizing portion and the speech recognizing portion, judging whether the care recipient is normal in health condition from an interaction with the care recipient; and in response to a judgment that the care recipient is abnormal, sending an abnormality report to the center equipment through the radio portion and the relay terminal to cause the center equipment to dispatch emergency men to the care recipient's home.

### BRIEF DESCRIPTION OF THE DRAWING

Further objects and advantages of the present invention will be apparent from the following description of the preferred embodiments of the invention as illustrated in the accompanying drawing, in which:
FIG. 1 is a schematic diagram showing an overall arrangement of a remote care service system in accordance with a first illustrative embodiment of the invention;
FIG. 2 is a perspective view of a stuffed doll (i.e., a wireless terminal disguised as a stuffed doll) 1 used in the remote care service system 100 of FIG. 1;
FIG. 3 is a schematic block diagram showing an arrangement of the body 2 of the stuffed doll or wireless terminal 1;
FIG. 4 is a partial illustration of the interior of a care recipient's home;
FIG. 5 is a flowchart showing an exemplary operation of the body 2 of the stuffed doll 1;
FIG. 6 is an exterior view of an exemplary wireless terminal in the form of a dog-type robot in accordance with a second illustrative embodiment of the invention;
FIG. 7 is a schematic block diagram showing an internal arrangement of the wireless terminal 1a of FIG. 6;
FIG. 8 is a diagram showing an exemplary structure of a time-phrase table for use in checking the health condition of a care recipient; and
FIG. 9 is a flowchart showing an exemplary operation of the wireless terminal 1a of FIG. 7.

Throughout the drawing, the same elements when shown in more than one figure are designated by the same reference numerals.

### DETAILED DESCRIPTION OF THE PREFERRED EMBODIMENTS

### Embodiment I

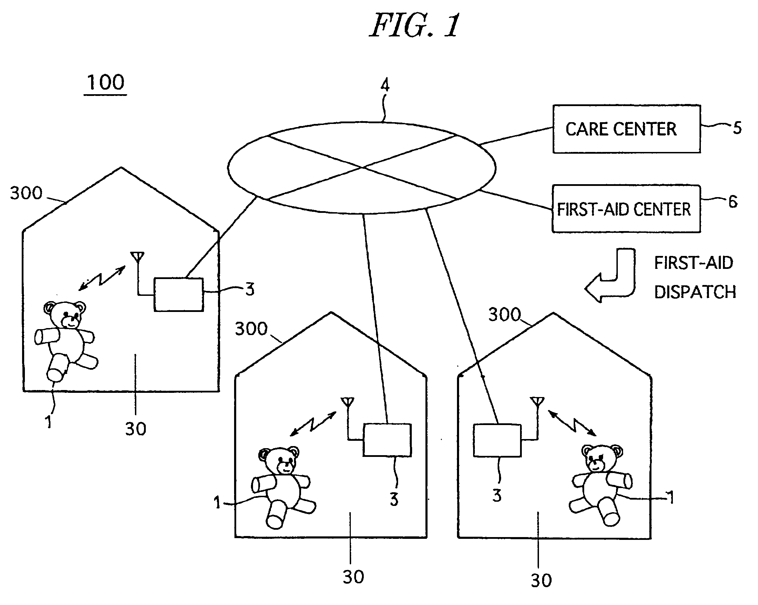

FIG. 1 shows an overall arrangement of a remote care service system in accordance with a first illustrative embodiment of the invention. In FIG. 1, remote care service system 100 comprises a plurality of home subsystems 30 provided within respective home 300 of each of the care recipients served by the system 100; a communication network 4, such as the Internet, which enables communications between each home subsystem 30 and other equipments constituting the system 100 as detailed later; a care center equipment 5 which receives an alert signal transmitted from a home subsystem 30 having detected an abnormal condition and responsively supervises the condition of a care recipient monitored by the alerting home subsystem 30; and a first-aid center equipment 6 which receives an emergency signal transmitted from a home subsystem 30 having detected an emergency condition. Each of the home subsystems 30 comprises at least one wireless terminal 1, disguised as a stuffed doll and placed in a room where the care recipient is living, for monitoring the care recipient positioned within a monitoring range thereof; and a communication or relay terminal 3 for radio-communicating with the terminal(s) 1 and communicating with care center equipment 5 and first-aid center equipment 6 via communication network 4. If the relay terminal 3 receives a wireless communication from a wireless terminal 1, then the relay terminal 3 establishes a dial-up call to, for example, an Internet service provider with which the care service provider has contracted in advance and communicates with care center equipment 5 or first-aid center equipment 6 via the Internet 4 (in this specific example). The home subsystems 30 are distributed by a care service provider to respective care recipients in contract with the care service provider.

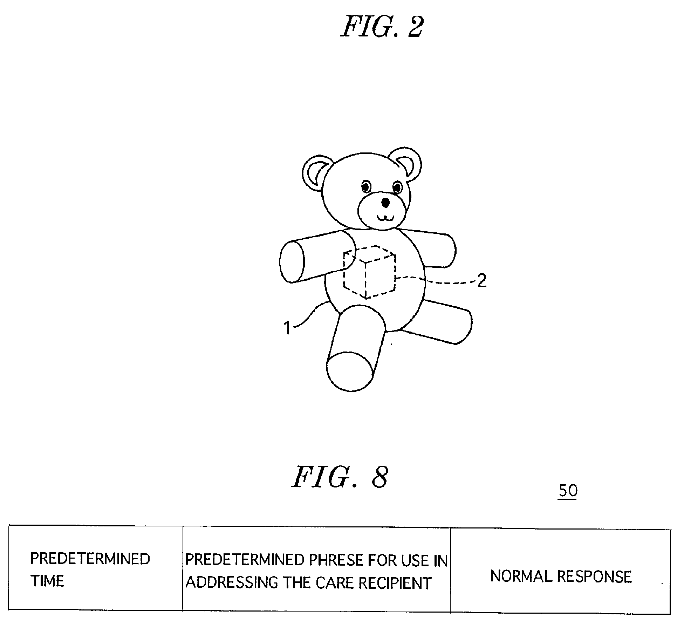

According to the first illustrative embodiment of the invention, each wireless terminal 1 is preferably disguised as any attachment-arousing and/or familiar article found in ordinary homes: e.g., a stuffed doll, a toy, a figure, a figurehead, a figurine, an ornament or the like. The article is such that at least gives a good impression to the care recipient. In this specific example, each wireless terminal 1 is realized as a stuffed doll within which the body 2 of the wireless terminal is installed as shown in FIG. 2. However, the body 2 of each wireless terminal 1 may be used as it is. In order to broaden the monitoring range, a plurality of wireless terminals 1 may be placed in a range permitting radio communications with communication or relay terminal 3 in a home.

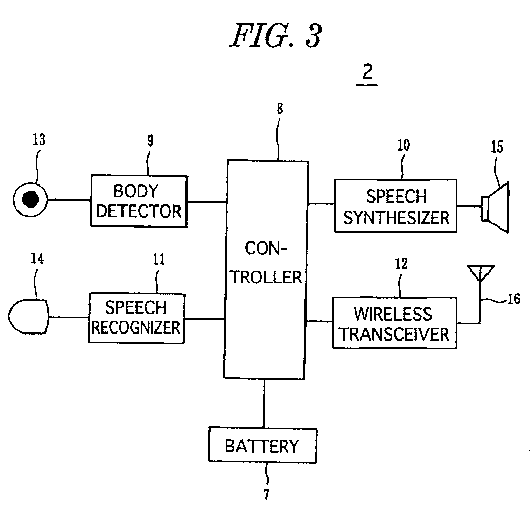

FIG. 3 is a schematic block diagram showing an arrangement of the body 2 of wireless terminal 1. In FIG. 3, the body 2 comprises
a battery 7 for supplying electric power to the other portions 8 through 14;
a controller 8 for controlling the entire body 2;
an infrared ray sensor 13 made up of a pyroelectric detector imbedded in, for example, an eyeball of the stuffed doll 1;
a human body detector 9 connected with infrared ray sensor 13 for detecting the presence of a human body on the basis of infrared rays radiated from the human body;
a speech synthesizer 10 for synthesizing a speech signal on the basis of stored speech patterns in response to an instruction given by the controller 8;
a loud speaker 15 for converting the speech signal from the speech synthesizer 10 into a speech;
a microphone 14 for converting an uttered speech into a speech signal;
a speech recognizer 11 for recognizing the speech signal from the microphone 14 to provide a recognition result to the controller 8; and
a wireless transceiver 12 for performing wireless communications between the controller 8 and the relay terminal 3.

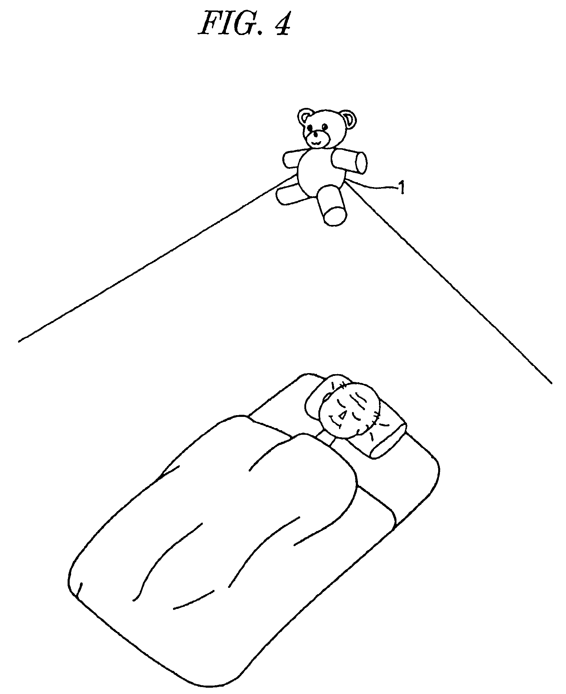

FIG. 4 is a partial illustration of the interior of a care recipient's home. In FIG. 4, the wireless terminal 1 is placed at a corner of the room where the care recipient is living or resting in bed. It is assumed that the monitoring range of the wireless terminal 1 is the entirety of the room and that since the care recipient is disabled in body, he or she is usually in bed or can move only within the room.

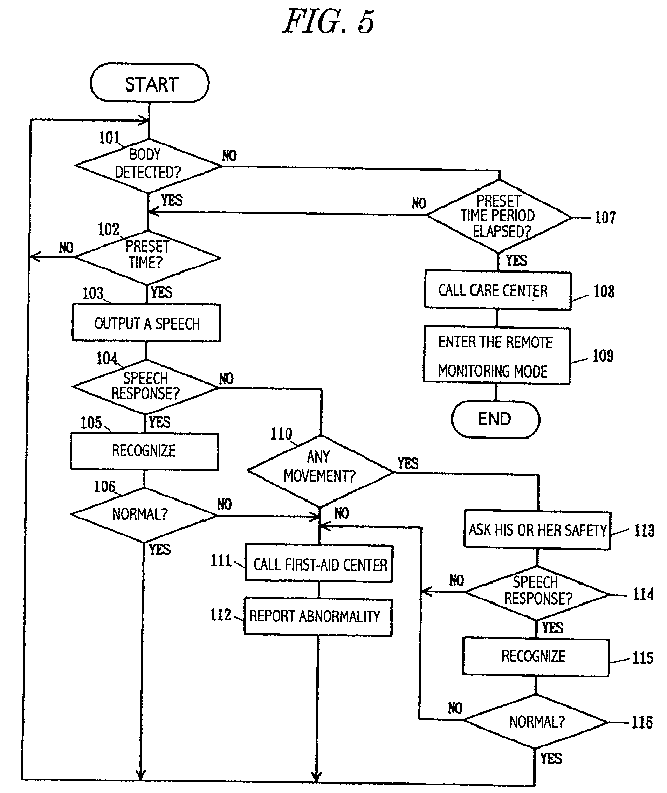

FIG. 5 is a flowchart showing an exemplary operation of controller 8 of wireless terminal 1 or the body 2 of wireless terminal 1. In step 101, controller 8 first makes a test to see whether a human body or the care recipient is found in the monitoring range (i.e., in the room).

If the care recipient is in the room, controller 8 can detect his or her presence from the detection of infrared rays from the care recipient. In this case (or if the answer is YES in step 101), controller 8 makes another test to see if it is one of predetermined times in step 102. If not, then controller 8 returns to step 101.

If it is one of predetermined times in step 102, then controller 8 addresses the care recipient by outputting a predetermined speech or phrase associated with the predetermined time in step 103.

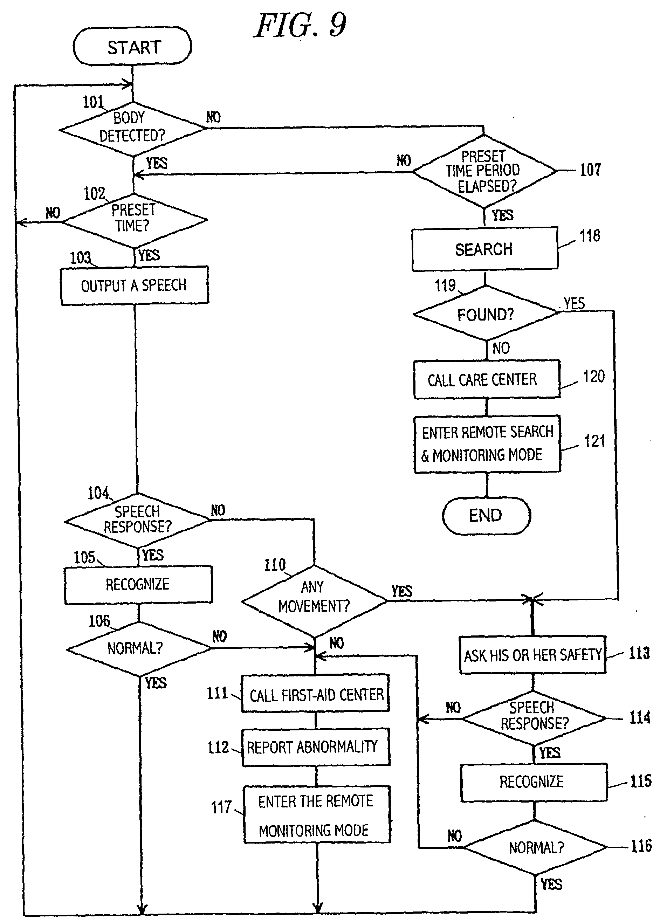

For this purpose, controller 8 stores, in not-shown memory, a time-phrase table as shown in FIG. 8. In FIG. 8, the time-phrase table 50 contains the predetermined times. For each predetermined time, the time-phrase table 50 contains a predetermined phrase for use in addressing the care recipient at the predetermined time and one or more normal (or usually expected) response for the predetermined phrase.

For example, if it is a predetermined time, say, 8:00 a.m., then controller 8 outputs a speech "Good morning, Mr. So-and-so!" associated with a time 8:00 a.m. in step 103. Then, in the following step 104, controller 8 makes a test to see if the care recipient returns any speech response. If so, then controller 8 recognizes the speech response in step 105, and makes a test in step 106 to see if the speech response is normal by comparing the speech response with the value in a NORMAL RESPONSE field of the record for 8:00 a.m. of the time-phrase table 50. The value will be "Good morning" for example in this specific case. If the speech response from the care recipient substantially accords with one of the values contained in the NORMAL RESPONSE field of the relevant record of the time-phrase table 50, then the controller 8 judges the speech response, i.e., the care recipient to be normal in step 106.

In the same way, at each of the predetermined times, controller 8 interactively judges whether the care recipient is normal by executing the steps 102 through 106.

If the test result is NO in step 106, i.e., if, though the care recipient can give some voice, he or she gives only an incomplete word such as a moan or utters some words other than predetermined normal response(s), which means that the care recipient is in a bad state of health, then controller 8 judges the care recipient to be abnormal in physical condition to establish a connection with the first-aid center equipment 6 through relay terminal 3 in step 111 and to report the abnormality to the first-aid center equipment 6 in step 112. Controller 8 makes the abnormality report by repeatedly transmitting the residential address and the name of the care recipient as well as a message to the effect of the abnormality. In response to a reception of the abnormality report, the person on duty in the first-aid center dispatches ambulance men to the home 300 identified by the received residential address. The remote care service system 100 may be configured such that, before dispatching the ambulance men, the person on duty is permitted to issue a command to cause the wireless terminal 1 to enter into a remote monitoring mode which enables conversation between the wireless terminal 1 and the first-aid center equipment 6. This mode enables the person on duty to obtain more information on the care recipient before ambulance men dispatching. After step 112, controller 8 returns to step 101.

If the test result is NO in step 104, i.e., the care recipient returns no response to the addressing by the wireless terminal 1, then since it is thought that the care recipient is either in a bad state of health or in the sleeping state, controller 8 makes a test in step 110 to see if any movement of the care recipient is detected from the detected infrared rays which changes in magnitude with a movement of the care recipient's body. If not, then controller 8 judges the care recipient to be abnormal in physical condition to proceed to the above-described step 111.

If any movement of the care recipient is detected in decision step 110, then controller 8 judges the care recipient to be sleeping to proceed to step 113, where controller 8 asks the care recipient's safety or give him/her a question "Mr. (or Ms.) So-and-so, are you all right?" for example. If the care recipient returns no response in step 114 or if, though the care recipient returns a response in step 114, a response from the care recipient is recognized in step 115 and judged to be abnormal in step 116, then controller 8 proceeds to the above-described step 111 to establish a connection with the first-aid center equipment 6 through relay terminal 3 in step 111 and to report the abnormality to the first-aid center equipment 6 in step 112, which results in the first-aid center 6 dispatching ambulance men to the home 300 provided with the abnormality reporting home subsystem 30.

If a human body can not be detected for a preset period of time, or if the test result is NO in step 101 and the test result is YES in step 107, which is the case when the care recipient falls senseless at the toilet for example, then controller 8 judges that the care recipient may become abnormal to establish a connection with the care center equipment 5 in step 108 and to enter into the remote monitoring mode in step 109. This mode enables the care worker of the care center 5 to remote control the wireless terminal 1. Specifically, the wireless terminal 1 in the remote monitoring mode outputs the voice uttered by the care worker of the care center 5 as it is. That is, if the care worker asks "Is Mr. So-and-so here" at the care center 5, then the wireless terminal 1 outputs a speech "Is Mr. So-and-so here" in the home 300. Conversely, since the wireless terminal 1 transmits a speech uttered by the care recipient to the care center equipment 5 as it is, the care worker of the care center can hear the speech uttered by the care recipient. which enables the care worker to monitor how the care recipient is.

If the care worker can not communicate with the care recipient even in the above-described remote monitoring mode, then the care worker in charge of the care center equipment 5 will call the first-aid center equipment 6 to request for the dispatch of ambulance men.

According to the first embodiment of the invention, the judgment of the care recipient's health condition is made interactively through communications with the care recipient and is, accordingly, more accurate than in the prior art system in which the judgment is made only based on data obtained by detection and measurement of the patient's medical condition.

Further, each wireless terminal 1 itself judges whether the care recipient is normal in health condition to make an abnormality report to either the care center or the first-aid center only when the care recipient is judged to be abnormal. In other words, the care worker of the care center has only to cope with an emergency in response to the abnormality report from a wireless terminal 1, enabling the care worker to serve a larger number of care recipients.

Also, since the body 2 of each wireless terminal 1 is installed in a stuffed doll, it can prevent the care recipients from feeling themselves being monitored, which facilitates the monitoring of the care recipients.

### Embodiment II

According to a second illustrative embodiment of the invention, the above-described wireless (monitoring) terminal function is installed in a movable or preferably walkable article such as various robots including a dog-type robot, a human-type robot, etc.

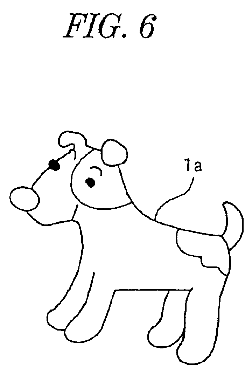

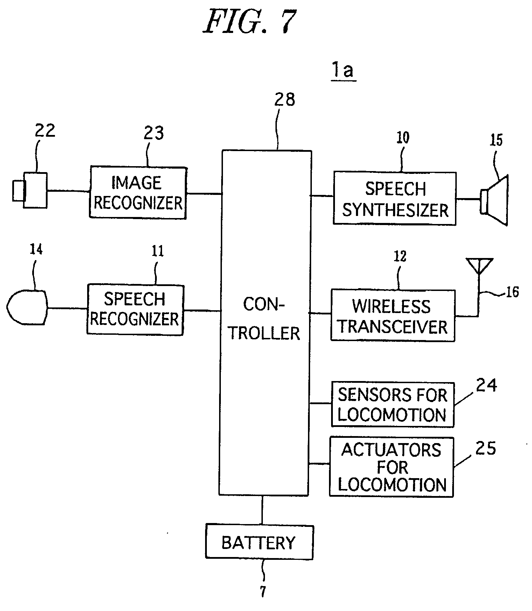

FIG. 6 is an exterior view of an exemplary wireless terminal in the form of a dog-type robot in accordance with the second illustrative embodiment of the invention. FIG. 7 is a schematic block diagram showing an internal arrangement of the wireless terminal 1a of FIG. 6. The wireless terminal 1a of FIG. 7 is identical to that of FIG. 3 except that sensors 24 and actuators 25 for locomotion have been added and the infrared sensor 13, the body detector 9 and the controller 8 has been replaced with a CCD (charge-coupled device) camera 22 for taking in an image of the monitor space, an image recognizer 23 for recognizing the care recipient and obstacles within home 300 in the taken-in image and a controller 28, respectively, in FIG. 7. Thus configured wireless terminal 1a can not only monitor a care recipient in the monitoring range in the above-described manner but also perform various actions that an ordinary dog-type robot can perform, including a walk with four legs, a lying down, a hand giving, etc.

FIG. 9 is a flowchart showing an exemplary operation of the wireless terminal 1a of FIG. 7. Since the flowchart of FIG. 9 is very similar to that of FIG. 5, only the different matters will be detailed in the following.

The wireless terminal 1a addresses 103 the care recipient at each predetermined time to judge 106 whether the care recipient is normal on the basis of the response by the care recipient in the same manner as in the first embodiment. However, if controller 28 judges the care recipient to be abnormal in any of the steps 106, 110, 114 and 116, then, after the above-described steps 111 and 112, controller 28 enters into the remote monitoring mode in step 117. Specifically, controller 8 outputs received speech signal from the first-aid center equipment 6 through the loud speaker 15 as speech; sends input speech signal from the microphone 14 via relay terminal 3 to the first-aid center equipment 6; and takes pictures of the care recipient by means of CCD camera 22 and sends the picture data to the first-aid center equipment 6. This enables a person in charge of the first-aid center 6 to cope with the situation in a more appropriate manner by better knowing the state of the care recipient from the received pictures.

Also, if controller 28 judges that the care recipient is absent for more than a predetermined period of time in step 107, then the wireless terminal 1a searches the interior of home 300 for the care recipient by using the image recognizer 23 in step 118. If the care recipient is found in step 119, then controller 28 proceeds to the above-described step 113.

If controller 28 can not find the care recipient in spite of the searching, then controller 28 establishes a connection with the care center equipment 5 in step 120 and enters into the remote search and monitoring mode in step 121. Specifically, the wireless terminal 1a enters into the remote monitoring mode as described in step 117 and searches the interior of home 300 for the care recipient by using the image recognizer 23. In this mode, the care worker of the care center 5 can examine the state of the interior of home 300 through the pictures taken by the wireless terminal 1a while remote controlling the wireless terminal or robot dog1a to walk avoiding obstacles.

According to the second embodiment of the invention, the capability of the care worker visually observing the remote care recipient through taken pictures and the capability of remote controlling the wireless terminal to moving (or walking) and searching the care recipient's home 300 for the care recipient enhances the reliability in the judgment of the recipient's health condition and expands the monitoring range of the remote care service system.

The foregoing merely illustrates the principles of the invention. Thus, many widely different embodiments of the present invention may be constructed without departing from the spirit and scope of the present invention.

For example, a single home subsystem 30 is used for each care recipient in the first embodiments. However, a plurality of home subsystems 30 may be used for a home 300.

In the second embodiment, a plurality of relay terminals 3 and a single wireless terminal 1a may be used for a home 300. This enables an effective expansion of the monitoring range of an inventive remote care service system.

In the second embodiment, the wireless terminal 1a is realized as a combination of hardware and software. It is also possible to realize an inventive wireless terminal 1a by adding an inventive wireless terminal program for providing the control of operation shown in FIG. 5 to an existing robot with a moving or walking ability.

In the above embodiments, if the abnormality is detected, an abnormality report is sent to the first-aid center 6. Instead of doing this, the abnormality report is first sent to the care center 5, which then sends a request for an ambulance man dispatch to the first-aid canter 6. Alternatively, the care center 5 and the first-aid center 6 may be incorporated into a single center.

It should be understood that the present invention is not limited to the specific embodiments described in the specification, except as defined in the appended claims.

## Claims

1. In a system comprising a center equipment capable of connecting with a communication network and a home subsystem which is provided in each home of a plurality of care recipients remote from the center equipment, each home subsystem comprising a wireless terminal having a radio communication capability and disguised as an article such as gives a good impression to the care recipient and relay means for relaying a communication between the wireless terminal and the center equipment, a method of providing care service to each of the care recipients, comprising the steps, executed by each wireless terminal, of:
judging whether the care recipient is normal in health condition from an interaction with the care recipient; and
in response to a judgment that the care recipient is abnormal, sending an abnormality report to the center equipment through the relay means to cause the center equipment to dispatch emergency men to the care recipient's home.

2. A method as defined in claim 1, wherein the wireless terminal further comprises means for moving to shift the position of the wireless terminal, a CCD camera for taking in an image of a monitor space of the wireless terminal and image recognition means for recognizing the care recipient and obstacles within the home in said image, the method further comprising the steps of:
in response to a judgment that the care recipient is absent for more than a predetermined period of time, searching the care recipient's home for the care recipient by using said image recognition means;
in response to a success in said searching, judging whether the care recipient is normal in health condition from a second interaction with the care recipient; and
in response to a judgment, from said second interaction, that the care recipient is abnormal, executing said step of sending an abnormality report.

3. A care recipient monitoring device for use in a remote care service system comprising a center equipment capable of connecting with a communication network and a home subsystem which is provided in each home of a plurality of care recipients remote from the center equipment to provide care service to each of the care recipients, wherein the care recipient monitoring device is included in each home subsystem which further comprises relay means for relaying a communication between the care recipient monitoring device and the center equipment, the care recipient monitoring device comprising:
means, constituting an enclosure of the care recipient monitoring device, for disguising the care recipient monitoring device as an article such as gives a good impression to the care recipient;
radio means for effecting a radio communication with the relay means;
judging means for judging whether the care recipient is normal through interaction with the care recipient; and
reporting means, responsive to a judgment that the care recipient is abnormal, for sending an abnormality report to the center equipment through said radio means and the relay means so as to cause the center equipment to dispatch emergency men to the care recipient's home.

4. A care recipient monitoring device as defined in claim 3, wherein said judging means includes means for taking account of whether the care recipient is absent in a monitoring space of the care recipient monitoring device for more than a predetermined period of time.

5. A care recipient monitoring device as defined in claim 3, wherein said judging means includes means for addressing the care recipient with a predetermined phrase at a predetermined time.

6. A care recipient monitoring device as defined in claim 5, wherein said judging means includes means, operated in the event that the care recipient nether orally answers to said predetermined phrase nor move at all, for judging that the care recipient is abnormal.

7. A care recipient monitoring device as defined in claim 5, wherein said judging means includes means, operated in the event that in response to said predetermined phrase the care recipient gives a voiced response different from one or more response associated with said predetermined phrase, for judging that the care recipient is abnormal.

8. A care recipient monitoring device as defined in claim 3, wherein the remote care service system further includes a second center attended by a care worker, said second center including first speech communication means for having a conversation with a person over said communication network and wherein the care recipient monitoring device further comprises:
second speech communication means for having a conversation with said care worker through said relay means, said communication network and said first speech communication means; and
means, operated in the event that the care recipient is absent in a monitoring space of the care recipient monitoring device for more than a predetermined period of time, for entering into an operation mode that enables said care worker to have a conversation with the care recipient.

9. A care recipient monitoring device as defined in claim 3, further comprising:
means for moving to shift the position of the care recipient monitoring device;
a CCD camera for taking in an image of a monitor space of the care recipient monitoring device;
image recognition means for recognizing the care recipient and obstacles within the home in said image;
means, responsive to a judgment that the care recipient is absent for more than a predetermined period of time, for searching the care recipient's home for the care recipient by using said image recognition means; and
second judging means response to a success in said searching for judging whether the care recipient is normal in health condition from a second interaction with the care recipient.

10. A care recipient monitoring device as defined in claim 9, wherein said center includes:
first speech communication means for having a conversation with a person over said communication network; and
video monitoring means for viewing said image, said center being attended by an attendant, and wherein the care recipient monitoring device further comprises:
second speech communication means for having a conversation with said care worker through said relay means, said communication network and said first speech communication means;
means for sending said image to said video monitoring means through said relay means and said communication network; and
said reporting means including means for entering into an operation mode that enables said attendant to have a conversation with the care recipient while viewing said image.

11. A care recipient monitoring device as defined in claim 9, wherein the remote care service system further includes a second center attended by a care worker and said second center is provided with:
first speech communication means for having a conversation with a person over said communication network; and
video monitoring means for viewing said image, and wherein the care recipient monitoring device further comprises:
second speech communication means for having a conversation with said care worker through said relay means, said communication network and said first speech communication means;
means for sending said image to said video monitoring means through said relay means and said communication network; and
means responsive to a failure in said searching for entering into an operation mode that enables said care worker to control the care recipient monitoring device to search said house for said care recipient while viewing said image in a state capable of having a conversation with said monitor space of the care recipient monitoring device.

12. A computer program recorded on a computer-readable medium and designed to operate a computerized device so as to provide a care service to a care recipient in a home remote from a center equipment in cooperation with a relay terminal installed in the home, wherein the relay terminal relays a communication between the computerized device and the center equipment which are linked with a communication network, the center equipment serves a multiplicity of care recipients and the computerized device comprises: computer means for controlling the entirety of the computerized device; radio means for effecting a radio communication with the relay terminal; speech recognizing means for recognizing a speech uttered by the care recipient; and speech synthesizing means responsive to a code from said computer means for outputting one of predetermined synthesized speech associated with said code, the computer program comprising the steps of:
through said speech synthesizing means and said speech recognizing means, judging whether the care recipient is normal in health condition from an interaction with the care recipient; and
in response to a judgment that the care recipient is abnormal, sending an abnormality report to the center equipment through said radio means and the relay terminal to cause the center equipment to dispatch emergency men to the care recipient's home.

13. A computer program as defined in claim 12, wherein the computerized device further comprises body detecting means for detecting a presence of a human body within a monitor range thereof; moving means for moving to shift the position of the computerized device, a CCD camera for taking in an image of a monitor space of the wireless terminal and image recognition means for recognizing the care recipient and obstacles within the home in said image, the computer program further comprising the steps of:
in response to a judgment based on said body detecting means that the care recipient is absent for more than a predetermined period of time, searching the care recipient's home for the care recipient by using said image recognition means and said moving means;
in response to a success in said searching, judging whether the care recipient is normal in health condition from a second interaction with the care recipient; and
in response to a judgment, from said second interaction, that the care recipient is abnormal, executing said step of sending an abnormality report.
